# EUROPEAN PATENT APPLICATION

(11) **EP 2 127 632 A1**
(43) Date of publication of application: **02.12.2009**
(21) Application number: 08157158.0
(22) Date of filing: 29.05.2008
(51) Int. Cl.: A61K 8/06, A61K 8/31, A61K 8/39, A61K 8/58, A61K 8/92, A61Q 13/00

(54) **Perfume composition with reduced alcohol content**

(71) Applicant: Coty Inc., New York, NY 10116 (US)
(72) Inventor: Bleuez, Loïc, 06700, Saint Laurent du Var (FR); Porcu, Maryse, 06240, Beausoleil (FR)
(74) Representative: Walter, Wolf-Jürgen

(57) **Abstract**

The invention relates to perfume compositions which are free of lower alcohols such as ethanol.

The composition comprises a transparent or translucent emulsion with an oil phase and a water phase wherein the oil phase comprises 25 to 95 wt-% of an oil mixture, selected from aromatic perfume oils and essential oils, and 5 to 75 wt-% of a non-aromatic solvent, the percentages relating to the total weight of the oil phase, and the solvent has a logP value of >5, and wherein the water phase comprises an emulsifier and a buffer system; the amount of the aromatic oils is in the range of 1 to 35 wt-%, related to the total weight of the perfume composition; and
the amount of monovalent C₂-C₅ alcohols in the oil phase or in the water phase or in both phases is in the range of 0 to 5 wt-% related to the total weight of the perfume composition; and
the water phase and the oil phase comprises further cosmetically acceptable ingredients.

## Description

### Field of Invention

The invention relates to perfume compositions which are free of lower alcohols such as ethanol.

### Background of the Invention

Lower alcohols, primarily ethanol, is the most interesting solvent of fragrance oil in perfumes. Ethanol evaporates quickly into the atmosphere, cools the skin and conveys a very desirable refreshing feeling on the human skin.

Regulations for environmental protection have recently become more stringent, also regarding volatile organic compounds. For this reason, one of the aims of the cosmetic industry is to reduce the high content of ethanol in perfumes or to replace it completely.

EP 0687460 B1 refers to low-alcohol perfume compositions comprising a lower glycol having up to six carbon atoms and/or a lower alcohol having four to six carbon atoms and at least four carbon atoms in its principal chain, and further comprising 40 - 75 per cent by weight of ethyl alcohol.

US 5,736,505 discloses a non-alcoholic fragrance comprising a hydrophobic perfume base, water, a non-ionic surfactant and glycereth-7 triacetate.

US 6,774,101 refers to an alcohol-free translucent perfuming composition with a surfactant of HLB > 10 being polyethylenglycol ethers and heavy paraffins. A preparation temperature of 75 - 95 °C is disadvantageous for that composition.

US 2007/0178144 refers to cosmetic O/W emulsions comprising at least one non-ionic primary emulsifier, at least one secondary emulsifier, one or more cosurfactants and usual cosmetic oils with the proviso that the water phase content of the emulsion is at least 70 wt-%.

The object of the present invention is to provide perfume compositions without lower alcohols but higher perfume oil contents in a long-lasting stable preparation and storable in a broad temperature range.

A further object is to provide a complete olfactory restitution of fragrance oil connected with a long-time olfactory stability.

A further object is to provide perfume compositions with conveying a fresh feeling and without stickiness on the skin.

### Summary of the Invention

The perfume composition of the invention with reduced alcohol content comprises a transparent, translucent or milky white emulsion with an oil phase and a water phase wherein
(a) the oil phase comprises 25 to 95 wt-% of an oil mixture of aromatic perfume oils and 5 to 75 wt-% of a non-aromatic solvent, the percentages relating to the total weight of the oil phase, and the solvent has a logP value of >5, and the solvent is selected from Isododecane, Isohexadecane, Isoeicosane, isoparaffine fluids, C₁₃-C₃₀ alkanes, Hydrogenated Didecene, Hydrogenated Didodecene, Hydrogenated Polydecene, Hydrogenated Polydodecene, Hydrogenated Tridodecene, Hydrogenated Polyisobutene, mineral oils, and a mixture of two ore more thereof, or the solvent is a linear silicone of the Dimethicone type, a cyclic silicone or a mixture thereof;
(b) the water phase comprises an emulsifier and a buffer system;
(c) the amount of the aromatic oils is in the range of 1 to 35 wt-%, related to the total weight of the perfume composition;
(d) the amount of monovalent C₂-C₅ alcohols in the oil phase or in the water phase or in both phases is in the range of 0 to 5 wt-% , related to the total weight of the perfume composition; and
(e) the water phase and the oil phase comprises further cosmetically acceptable ingredients.

The term "perfume composition" refers to a product of usual perfumes but also to other fragrances such as Eau de Cologne, pre shaves, after shaves, face waters, tonics etc.

"Perfume oils" refers to known substances for modifying the smell/odour of a product or providing a smell/odour to a person. Mixtures of natural essential oils or synthetic fragrances are possible.

The oil phase of the composition comprises perfume oils, essential oils or a mixture thereof. Essential oils which are smelling substances derived by physical processes, preferred distillations, from plants or spices are also subsumed in perfume oils.

Examples of perfume oils are extracts of flowers (lily, lavender, rose, jasmine, neroli, ylang-ylang), stems and leaves (geranium, patchouli, petitgrain), fruits (anis, coriander, cumin, juniper), fruit skins (bergamot, citrus, orange), roots (macis, angelica, cardamom, iris, calmus) wood (pine, sandalwood, guaiac, cedar, rose), herbs and grasses (tarragon, lemon grass, salvia, thyme), needles and branches (pine, fir), resins and balms (galbanum, elemi, benzol, myrrh, olibanum, opoponax). Further, animal raw materials such as zibet and castoreum. Typical synthetic fragrances are products from the ester type, ether type, aldehyde type, ketone type, alcohol type and hydrocarbon type.

Useful perfume oils include galaxolide (50 % of Isopropyl Myristate), Ethylene Brassylate, Hebanolide, Globanone, Musk Ketone, Musk Xylol, Trimofix, Sandalore, Boisambrene, Nerolidol, Cedramber, Iso E Super, Irisantheme, Damascone Alpha, Orange Bresil, Filial, Aldehyde Alpha Hexyl Cinnamic, Farnesol, Bacdanol, Ebanol, Guaiac Wood Acetate, Cedrenyl Acetate, Hexyl Salicylate, Verdox, Cis-3 Hexenyl Salicylate, Linalyl Acetate, Benzyle Salicylate and mixtures thereof. Other perfume oils are also possible according to Int. Cosmetic Ingredient Dictionary and Handbook, 12th ed. 2008, Vol. 3, p. 3193-3200 (edited by CTFA, Washington DC, USA).

To obtain a fragrance mixture with a combination of perfume oils it is preferred to complete the perfume oils by some aromatic substances such as alcohols e.g. benzylic alcohol, cis-3-hexenol, phenyl ethyl alcohol, anysil alcohol, isoamyl alcohol, 4 hexen-1-ol, phenoxyethanol; or other substances e.g. ethyl vanilline, vanilline, heliotropine, helional, coumarin, ethyl maltol, ethyl acetate, ethyl acetoacetate, 2-methyl pyrazine, linalool oxide; and also non-odour substances such as phenoxypropanediol, trimethyl-1,3-pentanediol, chlorphenesin, ethylhexyl glycerin, caprylyl glycol, glyceryl caprylate; and mixtures thereof. More generally it is preferred to complete with raw materials having a logP between 0.5 and 2.

The term "reduced alcohol content" means that the content of monovalent C₂-C₅ alcohols such as ethanol is in the range of 0 - 5 wt-%, preferably 0 - 2 wt-% and most preferably 0 wt-%.

It is also possible to add to the oils such substances as Isononyl Isononanoate, Octyldocenol, linear silicones of the Dimethicone type and cyclic silicones, Diethyl Hexyl Carbonate, Isopropyl Myristate, Ethylhexyl Palmitate, PG, DPG, TPG, DPPG, Ethyl Phtalate, Ethyl Hexyl Stearate, Decyl Cocoate, Dioctyl Carbonate, Cetearyl Ethyl Hexanoate, Decyl Oleate, Isocetyl Palmitate, Cetearyl Isononanoate, Myristyl Myristate, Hexyl Laurate, Isopropyl Isononanoate, Isopropyl Palmitate, Isopropyl Laurate, C12-15 Alkyl Benzoate, pentaerythritol esters (e.g. Pentaerythrityl Tetracaprylate/Tetracaprate or Dipentaerythrityl Pentaisononanoate), but this is not preferred.

The solvents in the oil phase preferably have a calculated log P value in the range 5 to 12, more preferable 5 to 9, where P stands for the octanol/water partitioning coefficient of a material, i.e. the ratio between its equilibrium concentration in octanol and water, which is a measure of hydrophobicity. High partitioning coefficient values are more conveniently given in the form of their logarithm to the base 10, log P. While log P values can be measured experimentally, and measured log P data is available for many perfumes, log P values are most conveniently calculated. There are several recognised calculation or estimation methods available commercially and/or described in the literature (see for example A. Leo, Chem. Rev. 93(4), 1281-1306, (1993), "Calculating log P oct from structures"). Generally these models correlate highly but may for specific materials produce log P values which differ in absolute terms (by up to 0.5 log units or even more). However, no one model is universally accepted as the most accurate across all compounds. This is particularly true for estimates on materials of high log P (about 5 or greater). In the present specification, calculated log P values are obtained using the estimation software of Syracuse Research Corp., SRC, which is well-known to the scientific community, and accepted as providing high-quality predictions of log P values. References to calculated log P values thus mean values obtained using the a.m. software.

Most preferred solvents are Isohexadecane (logP 7.79), C15-C19 Alkane, C13-C16 Isoparaffin (logP 7.63), and mixtures thereof. Other useful solvents are Isoeicosane (LogP 10.09), Isododecane (logP 6.16), paraffin oil ISO W 05.

Preferably the oil phase comprises 40 to 70 wt-% of the aromatic oil or oil mixture and 20 to 45 wt-% of the non-aromatic solvent, related to the total weight of the oil phase.

The amount of the aromatic oil is preferably in the range of 5 - 30 wt-%, especially 7 - 23 wt-%.

The emulsifier in the water phase is a non-ionic emulsifier or a mixture of non-ionic emulsifiers or preferred a mixture of a non-ionic emulsifier with an emulsifier or a mixture of emulsifiers having one or more acid functions.

Preferred non-ionic emulsifiers are selected from the group consisting of glyceryl partial esters, polyglyceryl partial esters, sorbitan partial esters, sorbitol partial esters, carbohydrate esters, (alkylpoly)glycosides and mixtures thereof. The used carboxylic acids and alcohols, respectively, are preferably C₆-C₂₂ carboxylic acids and C₆-C₂₂ alcohols, respectively. Examples are Polyglyceryl-2 Laurate, Polyglyceryl-3 laurate, Polyglyceryl-4 Laurate, Polyglyceryl-5 Laurate, Polyglyceryl-6 Laurate, Polyglyceryl-10 Laurate, Polyglyceryl-4 Dilaurate, Polyglyceryl-4 Stearate, Polyglyceryl-4 Isostearate, Polyglyceryl-4 Cocoate, Polyglyceryl-4 Oleate or Polyglyceryl-4 Caprate. Non-ionic emulsifiers with ethoxylated branches can also be used.

Preferred anionic emulsifiers in mixture with nonionics are e.g. sulfates, sulfonates, citrates, sulfosuccinates, succinates, tartrates and phosphates esterified with C₆-C₂₂ alcohols. Examples are dilauryl citrate or disodium lauryl sulfosuccinate. The anionic emulsifier can be neutralised with e.g. Na or K. Anionic emulsifiers with ethoxylated branches can also be used.

Special preferred emulsifiers are e.g. a mixture of polyglyceryl-4 laurate and dilauryl citrate (e.g. Tego® Care PL4 of Degussa) and dilauryl citrate and disodium lauryl sulfosuccinate (e.g. Rewopol® SB F 12 P of Degussa) or a mixture of Polyglyceryl-4 Laurate and disodium lauryl sulfosuccinate or a mixture of Polyglyceryl-3 Laurate (e.g. Hydramol® TGL Ester of Noveon) and Polyglyceryl-4 Laurate and Sorbitan Laurate (e.g. Tego® SML of Degussa) and Disodium Lauryl Sulfosuccinate.

The range of the solvent is usually 0.1 to 20 wt-%, preferably 0.5 to 10 wt-%, more preferred 2 to 10 wt-%, related to the total weight of the perfume composition.

The range of the emulsifier is usually 0.1 to 20 wt-%, preferably 0.5 to 12 wt-%, especially 0.5 to 2 wt-%. A further preferred range of the emulsifier is 3 to 10 wt-%.

The buffer system in the water phase plays a very important role for the stability of the whole emulsion composition.

By the buffer system the well-known pH drop which is described for aromatic substances due to, for example, some ester hydrolysis (Linalyle acetate, benzyl acetate or allyl amyl glycolate) or any aldehyde oxidation (Aldehyde α-hexyl cinnamic, helional or lilial).

The buffer system comprises at least one inorganic or organic component and preferably a mixture of components, inorganic and/or organic.

Special buffer systems are sodium dihydrogenphosphate / disodium hydrogenphosphate, citric acid/trisodium citrate, disodium citrate/HCl, potassium hydrogen phthalate/HCl, (potassium hydrogen phthalate/HCl)/NaOH, (disodium citrate/ HCl)/NaOH, potassium dihydrogen phosphate/disodium hydrogen phosphate, sodium barbital/HCl, borax solution/HCl , ((glycine+NaCl)/HCl)/NaOH, citric acid/disodium hydrogen phosphate, sodium acetate/acetic acid, imidazole/HCl, triethanol amine + titriplex III/HCl, tris(hydroxymethyl) aminomethane/HCl, sodium carbonate/sodium hydrogen carbonate and mixtures thereof.

Specially preferred are sodium dihydrogenphosphate/disodium hydrogenphosphate as well as citric acid/trisodium citrate.

Until now it has only been possible to formulate chemically stable aromatic perfume oils in water emulsions. It was almost impossible to obtain a stable nanoemulsion containing more than 0.1 wt-% of any buffer components. This refers to a one-month test in an oven at 45 °C, which is a usual stability test.

In this connection, the term "stable emulsion" means a particle size profile of the emulsion maintained for at least two months at room temperature. Analyses were made with the Dynamic Light Scattering (DLS) method, in which the coefficient of diffusion of the particles in solution was measured. Sample solutions were diluted by factor 10 with de-ionised water. The measuring took place by dynamic light scattering with a Malvern HPPS 3.1 at 25 °C for a duration of 100 seconds. The coefficient can be converted by the Stokes-Einstein equation to a mean hydrodynamic radius of the emulsion droplets (equal to particles).

The particle size of the droplets of the present invention are in the range of 5 nm to 1 µm; this means the oil droplets in the water phase or the water droplets in the oil phase. Preferred ranges are 5 to 200 nm, especially 10 to 50 nm.

The perfume composition of the present invention can be a milky white emulsion, a translucent emulsion or a transparent emulsion. The emulsion is preferably a translucent to transparent emulsion, more preferred a transparent emulsion with a transmission of more than 90 % to 100 %. Transparent emulsions have a particle diameter of 5 to 35 nm, translucent emulsions have a particle diameter of 35 to 95 nm.

The content of perfume oils and/or essential oils is in the range of 1 to 35 wt-%, preferably 5 - 20 wt-%, more preferred 7 - 15 wt-% related to the total weight of the perfume composition.

The present invention provides a technique for preparing a low-viscosity, low skin-irritating, fine and prolonged-stability emulsion containing a large amount of perfuming compound, as typically used for impregnating fragrance emulsions, sprayable low alcohol or alcohol-free perfuming emulsions or lotions.

Further important improvements are:
(1) Without the presence of lower alcohols or with largely reduced alcohol contents, a significantly better olfactory restitution of the fragrance's typical scent is possible, along with a longer lasting effective stability as compared to e.g. common Eau de Toilettes.
(2) The special selection of the components of the inventive perfume composition does not require high temperatures for the preparation of the emulsions, so that losses or partial losses of temperature-sensitive perfume oils or essential oils are avoided.
(3) Perfume compositions with high loaded with high loads of fragrance parts in transparent or translucent quality are possible.
(4) A long-term stability of the present invention's emulsions over the very broad temperature range from -20°C to +45°C/55°C was established in the accelerated aging test.
(5) The emulsions of the invention have a very good feeling to the skin and greatly reduce the feeling of stickyness due to the low content of surfactant.
(6) The emulsions are skin-friendly and provide only a very low irritation because of low surfactant concentrations.

The inventive preparation also contains further cosmetic auxiliary and carrier substances as they are used conventionally in such compositions, for example, water, floral waters, preservatives, colourings, chelating agent such as EDTA and the like, polyols (such as glycerine, polyglycols, PEG-400, sorbitol), UVA and UVB filters, gelling substances, tanning agents, polymers, copolymers, stabilisers and mixtures thereof, with the proviso that only substances for maintaining a translucent or transparent composition be selected.

The composition according to the invention can also advantageously contain antioxidants, quencher molecules and radical scavengers. Such substances include vitamins such as vitamin C and derivatives thereof, for example, ascorbic acetate, ascorbic phosphate, and ascorbic palmitate; vitamin A and derivatives thereof; folic acid and derivatives thereof; vitamin E and derivatives thereof such as Tocopheryl Acetate, Tocopheryl Linoleate, Tocopheryl Phosphate; flavones or flavonoids; amino acids, such as histidine, glycine, tyrosine, tryptophan, and derivatives thereof; carotenoids and carotenes such as α-carotene, β-carotene and mixtures thereof, with the same proviso as above. Well-known antioxidants are for example BHT, BHA, Tinogard® TT, Tinogard® TS, Tinogard® NOA (from Ciba), TBHQ, Propyl Gallate, Vitamin E TPGS (Tocophersolan), Parsol Guard® (from DSM) and various mixtures none under Covi-OX® (from Cognis) or Oxynex® (from MERCK); Quenchers as Tinoguard® Q (from Ciba), Spectrasolv® (from The Hallstar Company) or Oxynex® ST (from Merck).

The use of the cosmetic preparations according to the invention can be realized in the form of e.g. Eau de Toilettes, sun screen gels, after sun products, lotions, body gels, after shaves, pre shaves, Colognes, moisturizing Eau de Toilette.

The manufacture of such products is carried out in a way known to a person skilled in the art.

Below, the invention will be described in detail by examples. If not specified otherwise, all percentages are weight percentages.

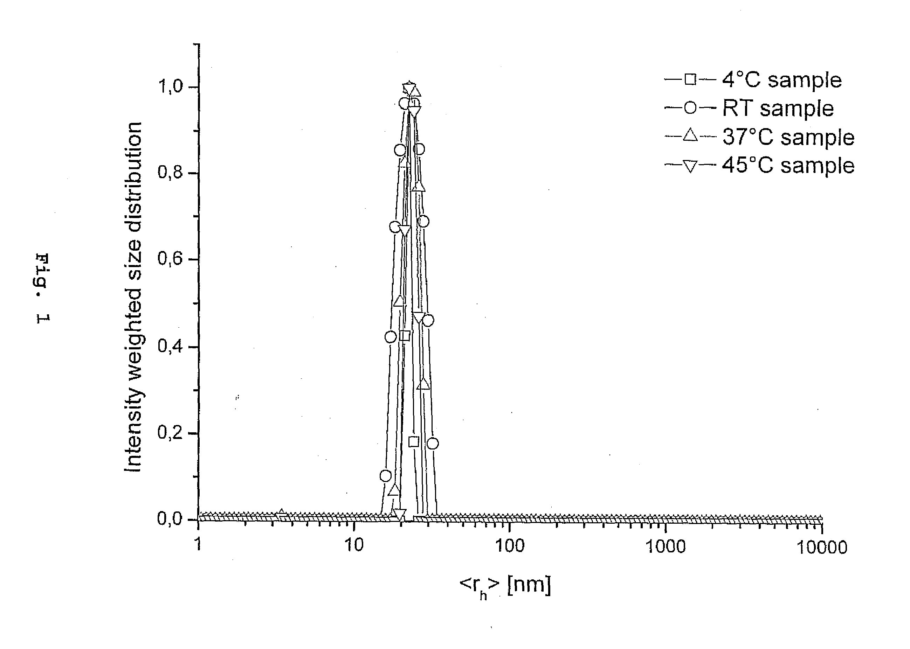

The accompanying drawings show
- Fig. 1: Exemplified graph with long-term stability of the emulsion according to Example 4 with intensity-weighted size distribution against the particle radius (r)

**Example 1 Perfume I**

| **Phase A** | |
|---|---|
| Water | 4.0 |
| Polyglyceryl-4 Laurate | 4.6 |
| Isohexadecane | 3.8 |
| Fragrance mixture with perfume oils (6.5) (inter alia Iso E Super, Lilial) and aromatic alcohols (1.5) (inter alia Benzyl Alcohol) | 8.0 |
| Glycerine | 2.5 |
| Disodium Lauryl Sulfosuccinate | 0.5 |

| **Phase B** | |
|---|---|
| Water | q.s. ad 100 |
| Sodium Dihydrogenphosphate | 0.8 |
| Disodium Hydrogenphsophate | 0.2 |

The ingredients of phase A are mixed at about 50°C with 350-450 rpm to reach a homogenous mixture. Phase B ingredients are mixed separately to dissolve the salts in the water. After that phase B is added slowly to phase A over about 15 - 30 minutes with an increasing speed of 350 to 1000 rpm. Homogenisation at 900 - 1000 rpm for 10 - 20 minutes is possible. Perfume I is a translucent emulsion.

**Example 2 Perfume II**

| **Phase A** | |
|---|---|
| Water | 4.4 |
| Polyglyceryl-4 Laurate | 4.6 |
| Isohexadecane | 3.8 |
| Fragrance mixture with perfume oils (6.0)**¹** and aromatic alcohols (1.5) | 7.5 |
| Glycerine | 2.5 |
| Disodium Lauryl Sulfosuccinate | 0.5 |

| **Phase B** | |
|---|---|
| Water | q.s. ad 100 |
| Sodium Dihydrogenphosphate | 0.8 |
| Disodium Hydrogenphsophate | 0.2 |

| | |
|---|---|
| ¹ Mixture of Galaxolide®, Iso E Super®, Habanolide®, Hedione®, Orange EO from Brazil, Dihydromyrcenol, Benzyle salicylate, Linalool, Linalyle acetate, Helional®, Alpha Damascone, Ald. Alpha Hexyl cinnamic. | |

The procedure is according to Example 1. Perfume II is a translucent emulsion.

**Example 3 Perfume III**

| **Phase A** | |
|---|---|
| Water | 15.0 |
| Polyglyceryl-4 Laurate | 16.0 |
| Isohexadecane | 13.5 |
| Fragrance mixture with perfume oils (24.5) (Perfect Match) and aromatic alcohols (5.5) (inter alia Phenoxyethanol) | 30.0 |
| Glycerine | 9.2 |
| Disodium Lauryl Sulfosuccinate | 1.5 |

| **Phase B** | |
|---|---|
| Water | q.s. ad 100 |
| Sodium Dihydrogenphosphate | 0.15 |
| Disodium Hydrogenphsophate | 0.05 |

The procedure is according to Example 1. Perfume III is a transparent emulsion.

**Example 4 Perfume IV**

| **Phase A** | |
|---|---|
| Water | 4.0 |
| Polyglyceryl-4 Laurate | 4.5 |
| Isohexadecane | 3.8 |
| Fragrance mixture with perfume oils (6.8) (Perfect Match) and aromatic alcohols (1.5) (inter alia Benzyl Alcohol | 8.3 |
| Glycerine | 2.5 |
| Disodium Lauryl Sulfosuccinate | 0.5 |

| **Phase B** | |
|---|---|
| Water | q.s. ad 100 |
| Sodium Dihydrogenphosphate | 0.8 |
| Disodium Hydrogenphsophate | 0.2 |

The procedure is according to Example 1. Perfume IV is a translucent emulsion.

The graph of Fig. 1 shows that samples with different storage temperatures of 4°C, 20°C, 37°C, and 45°C after eight weeks storage time have no alterations in the size distribution. This underlines the unique long-term stability of the emulsion. Additionally, an increased olfactory restitution of the fragrance's typical scent is to be noted.

**Example 5 Perfume V**

| **Phase A** | |
|---|---|
| Water | 4.0 |
| Polyglyceryl-4 Laurate | 4.6 |
| C13-C16 Isoparaffins | 4.0 |
| Fragrance mixture with perfume oils (7.0) (Perfect Match) and aromatic alcohols (1.5) | 8.5 |
| Glycerine | 2.5 |
| Disodium Lauryl Sulfosuccinate | 0.4 |

| **Phase B** | |
|---|---|
| Water | q.s. ad 100 |
| Sodium Dihydrogenphosphate | 0.8 |
| Disodium Hydrogenphsophate | 0.2 |

The procedure is according to Example 1. Perfume V is a translucent emulsion.

**Example 6 Perfume VI**

| **Phase A** | |
|---|---|
| Water | 6.0 |
| Polyglyceryl-4 Laurate | 4.5 |
| Isohexadecane | 3.8 |
| Fragrance mixture with perfume oils (6.5) and aromatic alcohols (1.5) | 8.0 |
| Glycerine | 3.0 |
| Disodium Lauryl Sulfosuccinate | 0.4 |

| **Phase B** | |
|---|---|
| Water | q.s. ad 100 |
| Sodium Dihydrogenphosphate | 0.75 |
| Disodium Hydrogenphsophate | 0.25 |
| Ethanol | 5.0 |

The procedure is according to Example 1. Perfume VI is a translucent emulsion.

**Example 7 Perfume VII**

| **Phase A** | |
|---|---|
| Water | 4.0 |
| Polyglyceryl-4 Laurate | 4.8 |
| Hydrogenated Polydecene | 3.9 |
| Fragrance mixture with perfume oils (6.5) and aromatic alcohols (1.5) | 8.0 |
| Glycerine | 3.0 |
| Disodium Lauryl Sulfosuccinate | 0.4 |

| **Phase B** | |
|---|---|
| Water | q.s. ad 100 |
| Sodium Dihydrogenphosphate | 0.8 |
| Disodium Hydrogenphsophate | 0.2 |

The procedure is according to Example 1. Perfume VII is a translucent emulsion.

**Example 8 Perfume VIII**

| **Phase A** | |
|---|---|
| Water | 1.0 |
| Polyglyceryl-4 Laurate | 0.8 |
| Isohexadecane | 0.6 |
| Fragrance mixture with perfume oils (1.0) and aromatic alcohols (0.3) (inter alia Benzyl Alcohol | 1.3 |
| Glycerine | 0.5 |
| Disodium Lauryl Sulfosuccinate | 0.1 |

| **Phase B** | |
|---|---|
| Water | q.s. ad 100 |
| Potassium Dihydrogenphosphate | 1.0 |
| Disodium Hydrogenphsophate | 0.3 |

The procedure is according to Example 1. Perfume VIII is a translucent emulsion.

**Example 9 Perfume IX**

| **Phase A** | |
|---|---|
| Water | 3.5 |
| Polyglyceryl-4 Laurate | 4.8 |
| Isohexadecane | 4.0 |
| Fragrance mixture with perfume oils (8.0) and aromatic alcohols (2.0) | 10.0 |
| Glycerine | 2 |
| Disodium Lauryl Sulfosuccinate | 0.5 |

| **Phase B** | |
|---|---|
| Water | q.s. ad 100 |
| Citric Acid | 0.1 |
| Trisodium Citrate | 0.9 |

The procedure is according to Example 1. Perfume IX is a translucent emulsion.

**Example 10 Perfume X**

| **Phase A** | |
|---|---|
| Water | 6.0 |
| Polyglyceryl-4 Laurate | 4.6 |
| Isohexadecane | 3.8 |
| Fragrance mixture with perfume oils (7.0) and aromatic alcohols (1.5) | 8.5 |
| Glycerine | 3.0 |
| Disodium Lauryl Sulfosuccinate | 0.4 |

| **Phase B** | |
|---|---|
| Water | q.s. ad 100 |
| Sodium Dihydrogenphosphate | 0.8 |
| Disodium Hydrogenphsophate | 0.2 |

The procedure is according to Example 1. Perfume X is a translucent emulsion.

**Example 11 Perfume XI**

| **Phase A** | |
|---|---|
| Water | 9.0 |
| Polyglyceryl-4 Laurate | 10.0 |
| Isohexadecane | 8.0 |
| Fragrance mixture with perfume oils (15) and aromatic alcohols (3.5) (inter alia Benzyl Alcohol) | 18.5 |
| Glycerine | 5.0 |
| Disodium Lauryl Sulfosuccinate | 1.0 |

| **Phase B** | |
|---|---|
| Water | q.s. ad 100 |
| Sodium Dihydrogenphosphate | 0.5 |
| Disodium Hydrogenphsophate | 0.1 |

The procedure is according to Example 1. Perfume XI is a translucent emulsion.

**Example 12 Perfume XII**

| **Phase A** | |
|---|---|
| Water | 7.0 |
| Polyglyceryl-4 Laurate | 8.0 |
| Cyclohexasiloxane & Cyclopentasiloxane | 6.5 |
| Isopropyl Myristate | 1.0 |
| Fragrance mixture with perfume oils (11) and aromatic alcohols (3) (inter alia Phenoxyethanol | 14.0 |
| Glycerine | 4.5 |
| Disodium Lauryl Sulfosuccinate | 1.0 |

| **Phase B** | |
|---|---|
| Water | q.s. ad 100 |
| Sodium Dihydrogenphosphate | 0.6 |
| Disodium Hydrogenphsophate | 0.2 |

The procedure is according to Example 1. Perfume XII is transparent.

**Example 13 Perfume XIII**

| **Phase A** | |
|---|---|
| Water | 4.0 |
| Polyglyceryl-4 Laurate | 4.5 |
| Isohexadecane | 3.8 |
| Fragrance mixture with perfume oils (6) and aromatic alcohols mixed with Helional (1.5) | 7.5 |
| Glycerine | 3 |
| Disodium Lauryl Sulfosuccinate | 0.4 |

| **Phase B** | |
|---|---|
| Water | q.s. ad 100 |
| Sodium Dihydrogenphosphate | 0.8 |
| Disodium Hydrogenphsophate | 0.2 |

The procedure is according to Example 1. Perfume XIII is milky white.

## Claims

1. Perfume composition with reduced alcohol content, comprising a transparent or translucent emulsion with an oil phase and a water phase wherein
(a) the oil phase comprises 25 to 95 wt-% of an oil mixture, selected from aromatic perfume oils and essential oils, and 5 to 75 wt-% of a non-aromatic solvent, the percentages relating to the total weight of the oil phase, and the solvent has a logP value of >5, and the solvent is selected from Isododecane, Isohexadecane, Isoeicosane, isoparaffine fluids, C13-C30 Alkane, Hydrogenated Didecene, Hydrogenated Didodecene, Hydrogenated Polydecene, Hydrogenated Polydodecene, Hydrogenated Tridodecene, Hydrogenated Polyisobutene, mineral oils, and a mixture of two or more thereof or the solvent is a linear silicone, a cyclic silicone or a mixture thereof;
(b) the water phase comprises an emulsifier and a buffer system;
(c) the amount of the aromatic oils is in the range of 1 to 35 wt-%, related to the total weight of the perfume composition;
(d) the amount of monovalent C₂-C₅ alcohols in the oil phase or in the water phase or in both phases is in the range of 0 to 5 wt-% , related to the total weight of the perfume composition; and
(e) the water phase and the oil phase comprises further cosmetically acceptable ingredients.

2. Perfume composition according to claim 1, wherein the oil phase comprises 40 to 70 wt-% of the aromatic oil or oil mixture and 20 to 45 wt-% of the non-aromatic solvent, related to the total weight of the oil phase.

3. Perfume composition according to claim 1, wherein the emulsifier is selected from a non-ionic emulsifier and a mixture of non-ionic emulsifiers and a mixture of a non-ionic emulsifier with an emulsifier having one or more acid functions.

4. Perfume composition according to claim 3, wherein the non-ionic emulsifier is selected from the group consisting of glyceryl partial esters, polyglyceryl partial esters, sorbitan partial esters, sorbitol partial esters, carbohydrate esters, (alkylpoly)glycosides and mixtures thereof, wherein the used carboxylic acids and alcohols are C₆-C₂₂ carboxylic acids and C₆-C₂₂ alcohols, respectively.

5. Perfume composition according to claim 3 wherein the anionic emulsifiers are sulfates, sulfonates, citrates, sulfosuccinates, tartrates and phosphates esterified with C₆ - C₂₂ alcohols or mixtures thereof.

6. Perfume composition according to claim 1, wherein the emulsifier is present in a range of 0.1 to 20 wt-%, preferably 0.5 to 12 wt-%, especially 0.5 to 2 wt-% and most preferably 3 to 10 wt-%, related to the total weight of the perfume composition.

7. Perfume composition according to claim 1, wherein the prefered emulsifier is a mixture of Polyglyceryl-4 Laurate and Disodium Lauryl Sulfosuccinate or Polyglyceryl-4 Laurate, Dilauryl Citrate and Disodium Lauryl Sulfosuccinate.

8. Perfume composition according to claim 1, wherein prefered solvent are Isohexadecane, Isoeicosane, C15-C19 Alkane, C13-C16 Isoparaffin and mixtures thereof.

9. Perfume composition according to claim 1, wherein the buffer system comprises at least one inorganic and one organic component.

10. Perfume composition according to claim 1, wherein the buffer system is selected from Sodium dihydrogenphosphate / Disodium hydrogenphosphate, Citric acid/Trisodium citrate, Disodium citrate/HCl, Potassium hydrogen phthalate/HCl, (Potassium hydrogen phthalate/HCl)/NaOH, (Disodium citrate/HCl)/NaOH, Potassium dihydrogen phosphate/Disodium hydrogen phosphate, Sodium barbital/HCl, Borax solution/HCl , ((Glycine+NaCl)/HCl)/NaOH, Citric acid/Disodium hydrogen phosphate, Sodium acetate/Acetic acid, Imidazole/HCl, Triethanol amine + Titriplex III/HCl, Tris(hydroxymethyl)aminomethane/HCl, Sodium carbonate/ Sodium hydrogen carbonate and mixtures thereof.

11. Perfume composition according to claim 1, wherein the buffer system is present in a range of 0.01 to 6 wt-%, related to the total weight of the perfume composition, preferably 0.4 to 3 wt-%, more preferred 0.4 to 1.5 wt-%.

12. Perfume composition according to claim 1, wherein the aromatic system contains an amount of raw materials having a LogP between 0.5 and 2 selected from alcohols of the group benzylic alcohol, cis-3-hexenol, phenyl ethyl alcohol, anysil alcohol, isoamyl alcohol, 4 hexen-1-ol, phenoxyethanol; and from the group of the other substances ethyl vanilline, vanilline, heliotropine, helional, coumarin, ethyl maltol, ethyl acetate, ethyl acetoacetate, 2-methyl pyrazine, linalool oxide; and the non-odour substances phenoxypropanediol, trimethyl-1,3-pentanediol, chlorphenesin, ethylhexyl glycerin, caprylyl glycol, glyceryl caprylate; and mixtures thereof.

13. Perfume composition according to claim 1, wherein the amount of monovalent C₂-C₅ alcohols is in the range of 0 to 2.0 wt-% , related to the total weight of the perfume composition, preferably 0 wt-%.

14. Perfume composition according to claim 1, wherein the oil phase further comprises linear silicones, cyclic silicones, Isononyl Isononanoate, Isopropyl Myristate, Ethylhexyl Palmitate or a mixture thereof.

15. Perfume composition according to claim 1, wherein the composition is a transparent or translucent composition with a particle diameter of 5 to 35 nm of transparent emulsions and a particle diameter of 35 to 95 nm of translucent emulsions.
